# EUROPEAN PATENT APPLICATION

(11) **EP 4 614 518 A1**
(43) Date of publication of application: **10.09.2025**
(21) Application number: 25162157.9
(22) Date of filing: 06.03.2025
(51) Int. Cl.: G16H 50/20, A61B 5/00

(54) **SYSTEMS AND METHODS FOR GENERATING QUANTIFIABLE EXPLANATION FOR MULTI-LEAD ELECTROCARDIOGRAM AND ASSOCIATED REGION OF INTEREST**

(30) Priority: 08.03.2024 IN 202421016966
(71) Applicant: Tata Consultancy Services Limited, Mumbai, Maharashtra 400 021 (IN)
(72) Inventor: PANDEY, Chandan, 700160 Kolkata, West Bengal (IN); DUTTA CHOUDHURY, Anirban, 700160 Kolkata, West Bengal (IN); KHANDELWAL, Sundeep, 201301 Noida, Uttar Pradesh (IN)
(74) Representative: Goddar, Heinz J.

(57) **Abstract**

Deep Learning (DL) performs well in cardiovascular disease (CVD) classification using 12-lead Electrocardiogram (ECG). However, explainable artificial intelligence in CVD classification still remains largely qualitative. Embodiments of the present disclosure provide systems and methods that implement a Region of Interest (ROI) based quantifiable explanation for multi-lead ECG. CVD specific post-processing steps are added, to increase the explanation performance. Furthermore, the system enables selection of an optimal DL model, within the performance space defined by classification, explanation, and time-complexity.

## Description

### CROSS-REFERENCE TO RELATED APPLICATIONS AND PRIORITY

The present application claims priority to Indian application no. 202421016966, filed on March 8, 2024.

### TECHNICAL FIELD

The disclosure herein generally relates to disease classification, and, more particularly, to systems and methods for generating quantifiable explanation for multi-lead electrocardiogram and associated region of interest.

### BACKGROUND

Deep Learning (DL) performs well in disease classification (e.g., cardiovascular disease (CVD)) classification using 12-lead Electrocardiogram (ECG). However, explainable artificial intelligence in CVD classification still remains largely qualitative, as the Deep Learning Models only provide the result without explaining why certain results were obtained. In high-stakes scenarios such as healthcare, the medical professionals do not completely trust the results generated by the models. They want more insight into why the model took a certain decision. Hence, researchers try to interpret the model's result using different tools. Thus, as mentioned above, explainability in disease classification (e.g., cardiovascular disease classification), remains largely qualitative, which means that the explanations provided are generally visual representations. The burden of finding the correctness of explanations is largely left to the person seeing the explanations.

### SUMMARY

Embodiments of the present disclosure present technological improvements as solutions to one or more of the above-mentioned technical problems recognized by the inventors in conventional systems.

For example, in one aspect, there is provided a processor implemented method for generating quantifiable explanation for multi-lead electrocardiogram and associated region of interest. The method comprises receiving, via one or more hardware processors, a multi-lead electrocardiogram (ECG) signal associated with a user; classifying, by using a trained classifier via the one or more hardware processors, the multi-lead ECG signal into one or more disease classes based on information present in each lead in the multi-lead ECG signal; generating, by using the trained classifier via the one or more hardware processors, an explainer model, based on an associated ECG training dataset; generating, by using the explainer model via the one or more hardware processors, an explanation comprising a contribution value to each data point present in each lead of the multi-lead ECG signal, wherein the contribution value is computed using the trained classifier and the multi-lead ECG signal; obtaining, via the one or more hardware processors, a filtered set of contribution values pertaining to a plurality of data points present in the multi-lead ECG signal based on a comparison of (i) the contribution value of the generated explanation assigned to each data point present in the multi-lead ECG signal, and (ii) a cut-off frequency range; normalizing, via the one or more hardware processors, the filtered set of contribution values pertaining to the multi-lead ECG signal, to obtain a set of normalized contribution values; determining, via the one or more hardware processors, one or more prominent ECG leads based on a comparison of (i) a mean contribution value computed for each lead present in the multi-lead ECG signal using the set of normalized contribution values and (ii) a threshold contribution value; analyzing, via the one or more hardware processors, a contribution of the one or more prominent ECG leads with respect to a reference lead contributing to a specific disease class amongst the one or more disease classes as classified by the trained classifier; and computing, via the one or more hardware processors, an explanation performance with respect to a ground-truth explanation using the analysed contribution of the one or more prominent ECG leads.

In an embodiment, the explanation performance is computed by categorizing the multi-lead ECG signal into a plurality of categories.

In an embodiment, the plurality of categories comprises a first category indicative of a presence of the one or more prominent ECG leads and the reference lead in the multi-lead ECG signal, a second category indicative of an absence of the one or more prominent ECG leads and the reference lead in the multi-lead ECG signal, a third category indicative of the presence of the one or more prominent ECG leads and the absence of the reference lead in the multi-lead ECG signal, and a fourth category indicative of the absence of the one or more prominent ECG leads and the presence of the reference lead in the multi-lead ECG signal.

In an embodiment, correctness of the generated explanation is measured based on number of one or more categories identified amongst the plurality of categories.

In an embodiment, a higher contribution value is assigned to the one or more prominent ECG leads in the multi-lead ECG signal which contributed to a classification of a disease to a desired disease class amongst the one or more disease classes.

In an embodiment, the filtered set of contribution values is obtained by applying a thresholding technique on the contribution value assigned to each data point present in the multi-lead ECG signal using the cut-off frequency range.

In an embodiment, the method further comprises identifying an associated region of interest (RoI) of the one or more prominent ECG leads based on an associated contribution value for computing the explanation performance.

In another aspect, there is provided a processor implemented system for generating quantifiable explanation for multi-lead electrocardiogram and associated region of interest. The system comprises: a memory storing instructions; one or more communication interfaces; and one or more hardware processors coupled to the memory via the one or more communication interfaces, wherein the one or more hardware processors are configured by the instructions to: receive a multi-lead electrocardiogram (ECG) signal associated with a user; classify, by using a trained classifier, the multi-lead ECG signal into one or more disease classes based on information present in each lead in the multi-lead ECG signal; generate, by using the trained classifier, an explainer model, based on an associated ECG training dataset; generate, by using the explainer model, an explanation comprising a contribution value to each data point present in each lead of the multi-lead ECG signal, wherein the contribution value is computed using the trained classifier and the multi-lead ECG signal; obtain a filtered set of contribution values pertaining to a plurality of data points present in the multi-lead ECG signal based on a comparison of (i) the contribution value of the generated explanation assigned to each data point present in the multi-lead ECG signal, and (ii) a cut-off frequency range; normalize the filtered set of contribution values pertaining to the multi-lead ECG signal, to obtain a set of normalized contribution values; determine one or more prominent ECG leads based on a comparison of (i) a mean contribution value computed for each lead present in the multi-lead ECG signal using the set of normalized contribution values and (ii) a threshold contribution value; analyze a contribution of the one or more prominent ECG leads with respect to a reference lead contributing to a specific disease class amongst the one or more disease classes as classified by the trained classifier; and compute an explanation performance with respect to a ground-truth explanation using the analysed contribution of the one or more prominent ECG leads.

In an embodiment, the explanation performance is computed by categorizing the multi-lead ECG signal into a plurality of categories.

In an embodiment, the plurality of categories comprises a first category indicative of a presence of the one or more prominent ECG leads and the reference lead in the multi-lead ECG signal, a second category indicative of an absence of the one or more prominent ECG leads and the reference lead in the multi-lead ECG signal, a third category indicative of the presence of the one or more prominent ECG leads and the absence of the reference lead in the multi-lead ECG signal, and a fourth category indicative of the absence of the one or more prominent ECG leads and the presence of the reference lead in the multi-lead ECG signal.

In an embodiment, correctness of the generated explanation is measured based on number of one or more categories identified amongst the plurality of categories.

In an embodiment, a higher contribution value is assigned to the one or more prominent ECG leads in the multi-lead ECG signal which contributed to a classification of a disease to a desired disease class amongst the one or more disease classes.

In an embodiment, the filtered set of contribution values is obtained by applying a thresholding technique on the contribution value assigned to each data point present in the multi-lead ECG signal using the cut-off frequency range.

In an embodiment, the one or more hardware processors are further configured by the instructions to identify an associated region of interest (RoI) of the one or more prominent ECG leads based on an associated contribution value for computing the explanation performance.

In yet another aspect, there are provided one or more non-transitory machine-readable information storage mediums comprising one or more instructions which when executed by one or more hardware processors cause generating quantifiable explanation for multi-lead electrocardiogram and associated region of interest by receiving a multi-lead electrocardiogram (ECG) signal associated with a user; classifying, by using a trained classifier, the multi-lead ECG signal into one or more disease classes based on information present in each lead in the multi-lead ECG signal; generating, by using the trained classifier, an explainer model, based on an associated ECG training dataset; generating, by using the explainer model, an explanation comprising a contribution value to each data point present in each lead of the multi-lead ECG signal, wherein the contribution value is computed using the trained classifier and the multi-lead ECG signal; obtaining a filtered set of contribution values pertaining to a plurality of data points present in the multi-lead ECG signal based on a comparison of (i) the contribution value of the generated explanation assigned to each data point present in the multi-lead ECG signal, and (ii) a cut-off frequency range; normalizing the filtered set of contribution values pertaining to the multi-lead ECG signal, to obtain a set of normalized contribution values; determining one or more prominent ECG leads based on a comparison of (i) a mean contribution value computed for each lead present in the multi-lead ECG signal using the set of normalized contribution values and (ii) a threshold contribution value; analyzing a contribution of the one or more prominent ECG leads with respect to a reference lead contributing to a specific disease class amongst the one or more disease classes as classified by the trained classifier; and computing an explanation performance with respect to a ground-truth explanation using the analysed contribution of the one or more prominent ECG leads.

In an embodiment, the explanation performance is computed by categorizing the multi-lead ECG signal into a plurality of categories.

In an embodiment, the plurality of categories comprises a first category indicative of a presence of the one or more prominent ECG leads and the reference lead in the multi-lead ECG signal, a second category indicative of an absence of the one or more prominent ECG leads and the reference lead in the multi-lead ECG signal, a third category indicative of the presence of the one or more prominent ECG leads and the absence of the reference lead in the multi-lead ECG signal, and a fourth category indicative of the absence of the one or more prominent ECG leads and the presence of the reference lead in the multi-lead ECG signal.

In an embodiment, correctness of the generated explanation is measured based on number of one or more categories identified amongst the plurality of categories.

In an embodiment, a higher contribution value is assigned to the one or more prominent ECG leads in the multi-lead ECG signal which contributed to a classification of a disease to a desired disease class amongst the one or more disease classes.

In an embodiment, the filtered set of contribution values is obtained by applying a thresholding technique on the contribution value assigned to each data point present in the multi-lead ECG signal using the cut-off frequency range.

In an embodiment, the one or more instructions which when executed by the one or more hardware processors further cause identifying an associated region of interest (RoI) of the one or more prominent ECG leads based on an associated contribution value for computing the explanation performance.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 depicts an exemplary system for generating quantifiable explanation for multi-lead electrocardiogram and associated region of interest, in accordance with an embodiment of the present disclosure.
FIG. 2 depicts an exemplary high level block diagram illustrating a method of training the system 100 of FIG. 1 for generating quantifiable explanation for multi-lead electrocardiogram (ECG) and associated region of interest, in accordance with an embodiment of the present disclosure, in accordance with an embodiment of the present disclosure.
FIG. 3 depicts an exemplary high level block diagram of the system of FIG. 1 for generating quantifiable explanation for multi-lead electrocardiogram and associated region of interest, in accordance with an embodiment of the present disclosure, in accordance with an embodiment of the present disclosure.
FIG. 4 depicts an exemplary flow chart illustrating a method for generating quantifiable explanation for multi-lead electrocardiogram and associated region of interest, using the systems of FIG. 1-2, in accordance with an embodiment of the present disclosure.
FIG. 5 depicts ECG Schematics; P, Q, R, S and T are the ECG waves; PR, ST, QRS, PR and QT are medically important ECG segments of a cardiac cycle, in accordance with an embodiment of the present disclosure.
FIGS. 6A and 6B, with reference to FIGS. 1 through 5, depict ST Segment Elevation (STE): SHapley Additive exPlanations (SHAP) time-series values with and without explanation and the black horizontal/spike line depicts the ECG signal, while the grey horizontal/spike line is SHAP of subject ID A0021, in accordance with an embodiment of the present disclosure.
FIGS. 7A and 7B depict first-degree atrioventricular block (IAVB): SHAP time-series values with and without explanation; the black horizontal spike line depicts the ECG signal, while the grey horizontal/spike line is SHAP; subject ID A0039, in accordance with an embodiment of the present disclosure.
FIGS. 8A and 8B depict SHAP spectrogram with and without explanation for STE, in accordance with an embodiment of the present disclosure.
FIGS. 9A and 9B depict SHAP spectrogram with and without explanation for IAVB, in accordance with an embodiment of the present disclosure.
FIGS. 10A and 10B depict qualitative results for STE for subject ID A0021, in accordance with an embodiment of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Cardiovascular diseases (CVDs) are the leading cause of premature death globally. It is estimated that around 17.8 million deaths in 2017 were caused by CVDs, and more than three quarters of CVD related deaths occurred in low income and middle-income countries. One of the primary reasons behind this is acute shortage of cardiologists. Many of these cardiologists work in urban areas, and rural patients are deprived of proper cardiac care. Hence, an early and accurate diagnosis of CVDs can significantly decrease mortality rates. Electrocardiogram (ECG) is the most common tool to diagnose CVDs and monitor heart conditions because of its non-invasive way to detect heartbeat signals. CVD is medically diagnosed, using 12-lead ECG. It is advantageous to use a 12-lead ECG over a single-lead ECG because it provides a more complete picture of the electrical activity of the heart. Abnormalities in ECG have also been associated with mortality risk predictors (e.g., refer "I. Mozos, A. Caraba et al., "Electrocardiographic predictors of cardiovascular mortality," Disease markers, vol. 2015, 2015."). Traditional ECG interpretation is a process that involves visually inspecting the ECG tracing and identifying specific features that are indicative of certain heart conditions. This can be a challenging task, even for experienced cardiologists. The advent of artificial intelligence (AI), especially in the field of deep learning (DL), enabled researchers to automatically interpret ECGs. This can be done much faster and, in some cases, more reliably, compared to traditional ECG interpretation. Currently, many commercial ECG analysis systems use conventional rule-base algorithms, to identify CVDs. These rules are encoded in medical guidelines and used by electrophysiologists for many years (e.g., refer "H. Blackburn, A. Keys, E. Simonson, P. Rautaharju, and S. Punsar, "The electrocardiogram in population studies: a classification system," Circulation, vol. 21, no. 6, pp. 1160-1175, 1960."). State of the art (SOTA) methods employ various machine learning methods on single-lead ECG (e.g., refer "A. Y. Hannun, P. Rajpurkar, M. Haghpanahi, G. H. Tison, C. Bourn, M. P. Turakhia, and A. Y. Ng, "Cardiologist-level arrhythmia detection and classification in ambulatory electrocardiograms using a deep neural network," Nature medicine, vol. 25, no. 1, pp. 65-69, 2019.", "A. Mukherjee, A. D. Choudhury, S. Datta, C. Puri, R. Banerjee, R. Singh, A. Ukil, S. Bandyopadhyay, A. Pal, and S. Khandelwal, "Detection of atrial fibrillation and other abnormal rhythms from ECG using a multilayer classifier architecture," Physiological measurement, vol. 40, no. 5, p. 054006, 2019.", and "H. Taniguchi, T. Takata, M. Takechi, A. Furukawa, J. Iwasawa, A. Kawamura, T. Taniguchi, and Y. Tamura, "Explainable artificial intelligence model for diagnosis of atrial fibrillation using holter electrocardiogram waveforms," International heart journal, vol. 62, no. 3, pp. 534-539, 2021."). With new medical datasets becoming available each year, 12-lead ECGs are also analyzed for automated CVD classification.

Embodiments of the present disclosure provide systems and methods that:
1. Define a protocol to quantify explanation. The protocol is based on comparing each point of reference lead/region of interest (RoI)/medical ROI (ground truth) against that of prominent lead/RoI as determined by the method of the present disclosure.
2. Add post-processing steps to SHapley Additive exPlanations (SHAP) to make it more efficient and readily usable by physicians. This contribution underlines the clinical significance of the method of the present disclosure.
3. Enable users to achieve qualitative explanation and quantifiable explanation.
4. Present two distinct use-cases, ST-segment elevation (STE) and first-degree atrioventricular block (IAVB), to present the enhanced quantifiable explainability offered by method of the present disclosure over conventional approach (SHAP).

Researchers have disclosed a three-layer machine learning (ML) network to classify rhythmic abnormalities in a noisy-single lead ECG classes. However, the classical ML is limited by scaling, as it is impractical to implement hand-crafted domain features for all CVDs. As the count of disease classes keeps on increasing, the shallow learning using handcrafting features becomes slower and less efficient. In order to mitigate this issue, most recent approaches employ DL in classification problems involving large number of classes. Hannun et al. used a 34-layer ResNet architecture classifying 12 CVDs using single-lead ECG. Ribeiro et al. used 12-lead ECG signal to classify six cardiac diseases, using a unidimensional ResNet architecture (e.g., refer "A. H. Ribeiro, M. H. Ribeiro, G. M. Paix~ao, D. M. Oliveira, P. R. Gomes, J. A. Canazart, M. P. Ferreira, C. R. Andersson, P. W. Macfarlane, W. Meira Jr et al., "Automatic diagnosis of the 12-lead ECG using a deep neural network," Nature communications, vol. 11, no. 1, p. 1760, 2020."). Chen et al. method secured first place in China Physiological Signal Challenge (CPSC) 2018 challenge, using an architecture made from a combination of CNN, RNN and attention layer (e.g., refer "T.-M. Chen, C.-H. Huang, E. S. Shih, Y.-F. Hu, and M.-J. Hwang, "Detection and classification of cardiac arrhythmias by a challenge-best deep learning neural network model," Iscience, vol. 23, no. 3, 2020."). In the CPSC challenge, the dataset provided by the organizers, hereinafter referred to as CPSC2018DB, contained 12-lead ECG representing combinations of nine cardiac diseases. Even though the DL architectures, proposed in SOTA methods, yield good performance, the main hindrance to use these models for practical purposes is its lack of explainability. These black-box models often fail to explain the reason behind their decisions. Therefore, the domain expert cannot comprehend the logical reasoning behind the AI's decision, let alone reviewing and aiding the decision-making AI.

Getting high performance in CVD classification is necessary, but not sufficient. Stakeholders involved in these decisions also need to know the explanation behind it. An explainable AI attempts to provide these explanations. Currently, various regulatory bodies are emphasizing the need for AI systems, especially in healthcare. For example, the European Union (EU) has published General Data Protection Regulation (GDPR), which empowers an individual to question why the AI system made a particular decision. In this section, we will present the xAI techniques used in CVD classification as well as telemedicine.

Local interpretable model-agnostic explanations (LIME) use interpretable surrogate models to explain complex nonlinear ML model (e.g., refer "M. T. Ribeiro, S. Singh, and C. Guestrin, "why should i trust you?" explaining the predictions of any classifier," in Proceedings of the 22nd ACM SIGKDD international conference on knowledge discovery and data mining, 2016, pp. 1135-1144."). Hughes et al. used LIME in CVD classification using ECG (e.g., "J. W. Hughes, J. E. Olgin, R. Avram, S. A. Abreau, T. Sittler, K. Radia, H. Hsia, T. Walters, B. Lee, J. E. Gonzalez et al., "Performance of a convolutional neural network and explainability technique for 12-lead electrocardiogram interpretation," JAMA cardiology, vol. 6, no. 11, pp. 1285-1295, 2021."). In game theory, Shapley values calculate the contribution of an individual player in a game. SHapley Additive exPlanations (SHAP) is a popular xAI technique which employs the usage of shapely values in determining the explanation (e.g., refer "S. M. Lundberg and S.-I. Lee, "A unified approach to interpreting model predictions," in Advances in Neural Information Processing Systems, I. Guyon, U. V. Luxburg, S. Bengio, H. Wallach, R. Fergus, S. Vishwanathan, and R. Garnett, Eds., vol. 30. Curran Associates, Inc., 2017."). Zhang et al. and Wickramsinghee et al. use SHAP to identify the contribution of input features in prediction of a class (e.g., refer "D. Zhang, S. Yang, X. Yuan, and P. Zhang, "Interpretable deep learning for automatic diagnosis of 12-lead electrocardiogram," Iscience, vol. 24, no. 4, 2021.", and "N. L. Wickramasinghe and M. Athif, "Multi-label classification of reduced-lead ecgs using an interpretable deep convolutional neural network," Physiological Measurement, vol. 43, no. 6, p. 064002, 2022."). Grad-CAM (e.g., refer "B. Zhou, A. Khosla, A. Lapedriza, A. Oliva, and A. Torralba, "Learning deep features for discriminative localization," in Proceedings of the IEEE conference on computer vision and pattern recognition, 2016, pp. 2921-2929.") has been used to highlight regions in the ECG signal which played an essential role in prediction of the class.

General practitioners (GP) and nurses may lack the necessary skill to interpret ECG for complex CVDs. Thus, it is important to provide access to telemedicine with reliable explanations in order to save time, cost, and lives. Lee et al. co-designed a machine learning-based decision support system for stroke rehabilitation assessment (e.g., "M. H. Lee, D. P. Siewiorek, A. Smailagic, A. Bernardino, and S. Berm'udez i Badia, "A human-ai collaborative approach for clinical decision making on rehabilitation assessment," in Proceedings of the 2021 CHI conference on human factors in computing systems, 2021, pp. 1-14."). Jacobs et al. also designed an AI decision support system for selection of treatment for major depressive disorders (e.g., refer "M. Jacobs, J. He, M. F. Pradier, B. Lam, A. C. Ahn, T. H. McCoy, R. H. Perlis, F. Doshi-Velez, and K. Z. Gajos, "Designing ai for trust and collaboration in time-constrained medical decisions: a sociotechnical lens," in Proceedings of the 2021 chi conference on human factors in computing systems, 2021, pp. 1-14."). However, these methods employ interviews with physicians, which can be time-consuming and costly. Lucic et al. developed an AIsystem for ECG noise detection using saliency maps, to help technicians to identify low-quality ECG exams (e.g., refer "A. Lucic, S. Ahmad, A. F. Brinhosa, V. Liao, H. Agrawal, U. Bhatt, K. Kenthapadi, A. Xiang, M. de Rijke, and N. Drabowski, "Towards the use of saliency maps for explaining low-quality electrocardiograms to end users," arXiv preprint arXiv:2207.02726, 2022."). Saliency maps helps in identifying the macro problems in ECG, but it is less effective for micro problems such as providing clear explanations for various complex heart conditions, where the pattern varies amongst the condition and the pattern lies in very brief time interval (as low as 50ms).

Embodiments of the present disclosure provide systems and methods that implement a Region of Interest (ROI) based quantifiable explanation for multi-lead ECG. CVD specific post-processing steps are added, to increase the explanation performance. Furthermore, the system enables selection of an optimal DL model, within the performance space defined by classification, explanation, and time-complexity.

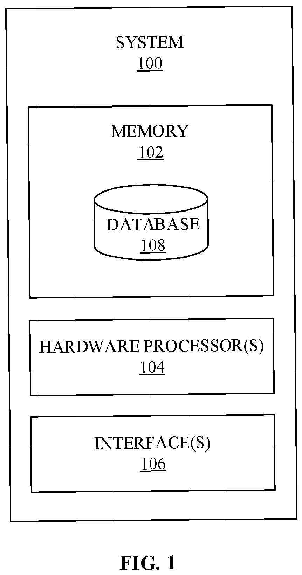

Referring now to the drawings, and more particularly to FIGS. 1 through 10B, where similar reference characters denote corresponding features consistently throughout the figures, there are shown preferred embodiments, and these embodiments are described in the context of the following exemplary system and/or method.

FIG. 1 depicts an exemplary system 100 for generating quantifiable explanation for multi-lead electrocardiogram and associated region of interest, in accordance with an embodiment of the present disclosure. In an embodiment, the system 100 may also be referred to as 'explanation generation system' and may be interchangeably used herein. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is/are configured to fetch and execute computer-readable instructions stored in the memory. In an embodiment, the system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random-access memory (SRAM) and dynamic-random access memory (DRAM), and/or non-volatile memory, such as read only memory (ROM), erasable programmable ROM, flash memories, hard disks, optical disks, and magnetic tapes. In an embodiment, a database 108 is comprised in the memory 102, wherein the database 108 comprises information pertaining to one or more multi-lead electrocardiogram (ECG) signals associated with one or more users, one or more disease classes, explanations and associated contribution values pertaining to each lead in the one or more multi-lead electrocardiogram (ECG) signals, and the like. The database 108 further comprises one or more ECG training dataset(s), one or more filtered sets of contribution values obtained based on cut-off frequency range, one or more sets of normalized contribution values, prominent ECG lead(s) identified/determined as a region of interest (RoI) based on threshold contribution value, analysed contribution of the prominent ECG lead serving as the RoI with respect to a reference RoI, and the like. The database 108 further stores explanation performance with respect to a ground-truth explanation using the analysed contribution of the prominent ECG lead serving as the RoI, various categories wherein number of categories for a given ECG lead signal indicate correctness of explanation being measured, and the like. The memory 102 stores one or more models such as but are not limited to a classifier/classification model, an explainer model, and the like which when executed enable the system 100 to perform the method described herein. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

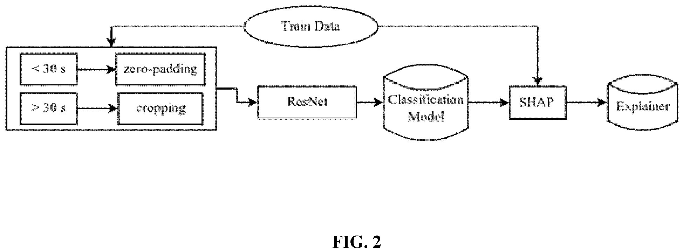

FIG. 2, with reference to FIG. 1, depicts an exemplary high level block diagram illustrating a method of training the system 100 of FIG. 1 for generating quantifiable explanation for multi-lead electrocardiogram and associated region of interest, in accordance with an embodiment of the present disclosure, in accordance with an embodiment of the present disclosure.

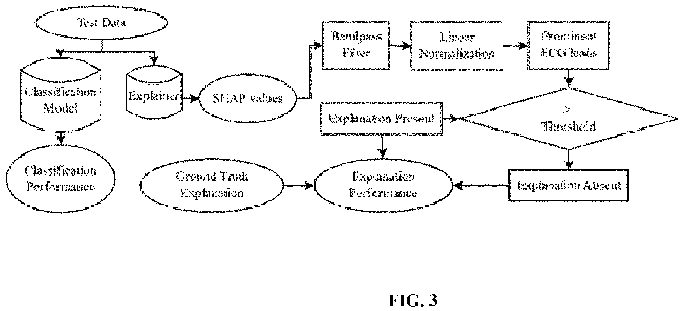

FIG. 3, with reference to FIG. 1, depicts an exemplary high level block diagram of the system 100 of FIG. 1 for generating quantifiable explanation for multi-lead electrocardiogram and associated region of interest, in accordance with an embodiment of the present disclosure, in accordance with an embodiment of the present disclosure.

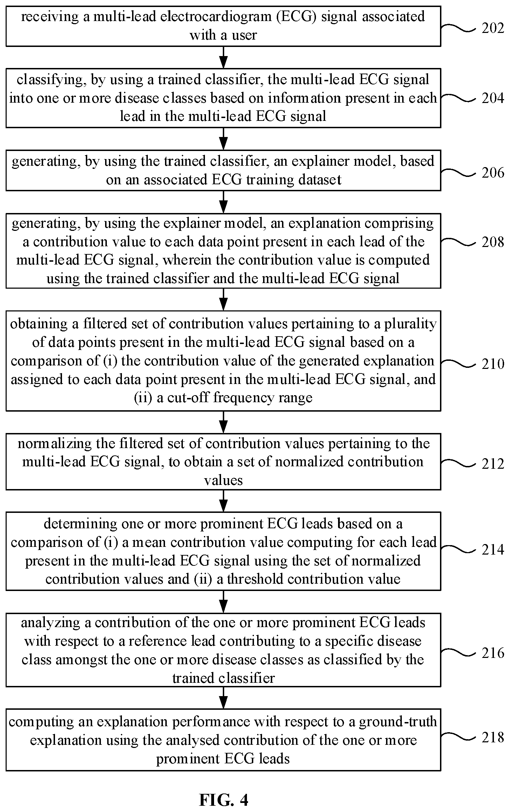

FIG. 4, with reference to FIGS. 1-3, depicts an exemplary flow chart illustrating a method for generating quantifiable explanation for multi-lead electrocardiogram and associated region of interest, using the systems 100 of FIG. 1-2, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to components of the system 100 of FIG. 1, the block diagram of the system 100 depicted in FIGS. 2-3, and the flow diagram as depicted in FIG. 4. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

At step 202 of the method of the present disclosure, the one or more hardware processors 104 receive a multi-lead electrocardiogram (ECG) signal associated with a user. In the present disclosure, the multi-lead electrocardiogram (ECG) signal was obtained from a publicly available dataset (e.g., China Physiological Signal Challenge (CPSC): CPSC2018DB). The dataset consists of 'x' records (e.g., 6877) ranging between p-q seconds (e.g., 6-60 seconds), at a sampling rate of 'y' Hertz (e.g., 500 Hz). It is a multi-label multi-class dataset with multi-lead ECG signal (e.g., 12-lead ECG signal). CPSC2018DB contains nine types of ECG signals. Depending on the requirement and application of the system 100 and method, any other lead signal (1 lead, 2-lead ECG signals, EEG signal, PPG signal, and so on) may be used as input to the system 100 to perform various disease classification (e.g., heart disease, neurodegenerative disease, and so on) and the method described herein.

Once, the multi-lead ECG signal is received, at step 204 of the method of the present disclosure, the one or more hardware processors 104 classify, by using a trained classifier (e.g., as known in the art classifier (or classification model), the multi-lead ECG signal into one or more disease classes (as known in the art disease classes) based on information present in each lead in the multi-lead ECG signal. The classifier is trained by receiving ECG training data comprising 12-lead ECG signal from a database (e.g., the CPSC dataset: CPSC2018DB). Further, the system 100 pre-processes the 12-lead ECG signal to obtain a predefined signal length (e.g., 30 seconds). For instance, If the signal length is less than a predefined signal length (e.g., 30 seconds), then zero padding is performed to obtain predefined signal length. If the signal length is greater than predefined signal length (30 seconds), then the signal may be randomly cropped to obtain the signal of the predefined signal length (e.g., 30 seconds). Once the cropped or padded signal is obtained of specific length (e.g., the pre-processed 12-lead ECG signal), the classifier is generated/obtained by training a deep learning network (e.g., a residual neural network (ResNet)). The trained ResNet is referred to as the classifier (or a classification model), in the present disclosure. FIG. 2 depicts the training of ResNet to obtain the trained classifier. The above step of obtaining the trained classifier is better understood by way of following description:

The system 100 and the method of the present disclosure train a multi-class classifier. As mentioned above, the ECG signals in CPSC2018DB vary in length. A homogenous number of data points across the dataset is therefore required to train a deep network architecture. A domain expert often requires at least 30 seconds of ECG data to reach a conclusion. Hence, shorter ECG signals are zero padded and longer signals are trimmed randomly to ensure 30 seconds length for each sample. This 1D ECG having 15000 data points (500Hz x 30 seconds) is the input to the deep architecture. ResNet is an ensemble of 130 models. Traditionally, 1D-ResNet34 architecture with four residual blocks has proven to outperform other single model pipelines (e.g., refer "D. Zhang, S. Yang, X. Yuan, and P. Zhang, "Interpretable deep learning for automatic diagnosis of 12-lead electrocardiogram," Iscience, vol. 24, no. 4, 2021."). The system 100 reuses this 1D-ResNet34 architecture wherein associated hyperparameters used in the model (ResNet) are the following. Adam optimizer and cross-entropy have been used as the optimization method and loss function respectively. The batch size, the maximum number of epochs, the learning rate and the dropout are 32, 30, 10⁻⁴ and 0.2 respectively. The system 100 also employed a two-residual block version of this architecture. Depending on the requirement and application of the system 100 and method, any deep learning model/machine learning model may be trained to obtain as the trained classifier for performing the step 204.

Once the trained classifier is obtained, at step 206 of the method of the present disclosure, the one or more hardware processors 104 generate, by using the trained classifier, an explainer model, based on an associated ECG training dataset. In an embodiment of the present disclosure, the explainer model is configured to estimate contribution of each data point present in the multi-lead ECG signal in predicting the one or more disease classes.

At step 208 of the method of the present disclosure, the one or more hardware processors 104 generate, by using the explainer model, an explanation comprising a contribution value to each data point present in each lead of the multi-lead ECG signal. In an embodiment, the contribution value (also referred to as contribution and interchangeably used herein) is computed using the trained classifier and the multi-lead ECG signal. In one embodiment, a higher contribution value is assigned to the one or more prominent ECG leads in the multi-lead ECG signal which contributed to a classification of a disease to a desired disease class amongst the one or more disease classes. Such assignment of the contribution value is performed by the explainer model and may vary depending on the application and requirement of the system 100 being implemented in one or more domains (e.g., healthcare). Such assignment of the contribution value shall not be construed as limiting the scope of the present disclosure. From a single sample, a 12-lead ECG signal is obtained. Each lead has 15000 data points (30 secs). Thus, from a single sample input data of size 12 x 15,000 = 180,000 data points is obtained. There are a total nine disease classes that the model is classifying. The explainer model estimates the contribution of each data point towards each class. Thus, for a single data point, contribution values are towards 9 different classes. So, for a single data point, the explainer model outputs a vector of length 9. Each value in the vector represents contribution values towards each class. An example of that vector is as illustrated below:
[-3.97668302e-05, 5.89934640e-05, 1.15100549e-04, -1.70589179e-05, - 2.72599744e-05, -1.37509582e-07, 3.28795843e-05, -6.81474675e-05, -7.49984334e-05]

The above steps 206 and 208 are better understood by way of following description:

The main aim of the system 100 of the present disclosure is to quantify the explanation methods in general and implement the method of FIG. 4 to improve the quantification. The system 100 chose SHAP (SHapley Additive exPlanations) over LIME (Local interpretable model-agnostic explanations) as it is built on the ideology of LIME and claimed a better performance compared to LIME. SHAP is generated for each input data point of ECG time series data. The contribution of each input ECG signal point is calculated for each class. Thus, for a 500Hz 12-lead ECG, where the duration of each lead is 30 sec (15000 data points), the SHAP contributions of this 12×15000 data points for each of nine classes are calculated. The final SHAP matrix has a dimension of 9×12×15000. From the 12 ECG leads, only those lead who's mean SHAP value crosses a heuristic and pre-defined threshold (10⁻⁴) (also referred to as threshold contribution value or threshold or threshold value and interchangeably used herein), are selected.

At step 210 of the method of the present disclosure, the one or more hardware processors 104 obtain a filtered set of contribution values pertaining to a plurality of data points present in the multi-lead ECG signal based on a comparison of (i) the contribution value of the generated explanation assigned to each data point present in the multi-lead ECG signal, and (ii) a cut-off frequency range. In one embodiment, the filtered set of contribution values is obtained by applying a thresholding technique on the contribution value assigned to each data point present in the multi-lead ECG signal using the cut-off frequency range. For instance, the threshold technique may include applying a bandpass filter on the contribution value, in one embodiment of the present disclosure.

The above step of obtaining the filtered set of contribution values is better understood by way following description:

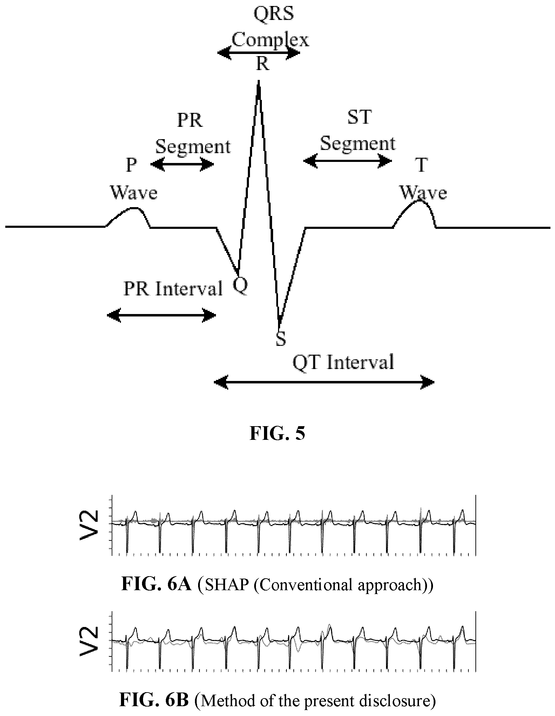

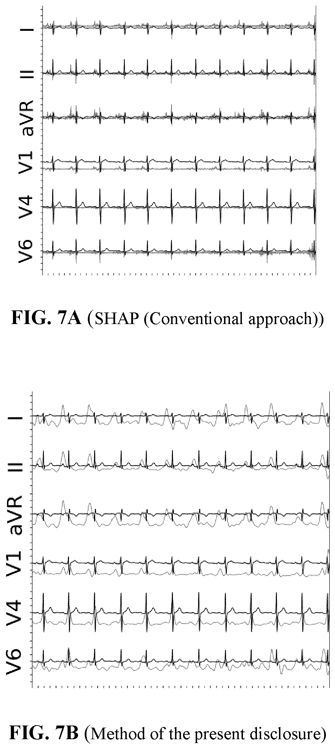

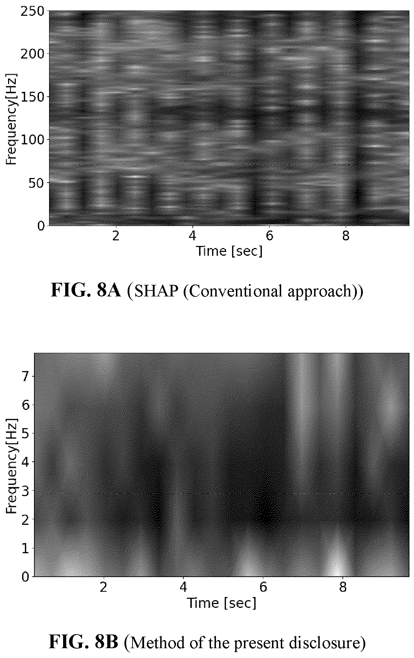

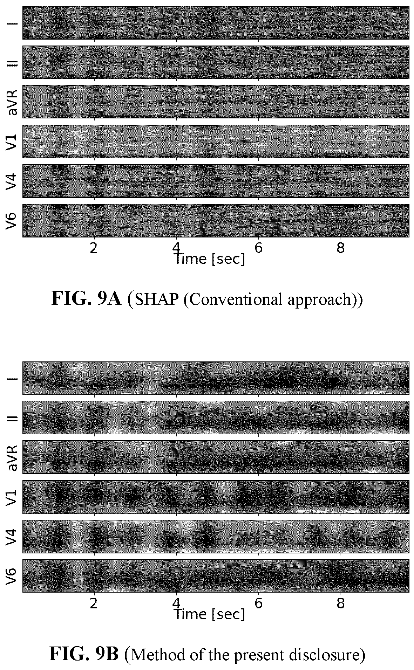

Firstly, the system 100 demonstrates explainability in context of the CVDs present in the CPSC database CPSC2018DB. Then, the system 100 quantifies the explanations. Finally, the system 100 introduces ROI based post-processing steps to enhance the explanation.
1. RoI (region of interest) based Post-Processing: Only specific segments of the cardiac cycle are medically significant in capturing a specific disease. The entirety of the cardiac signal is neither required nor can be explained as medical RoI (e.g., also referred to as reference RoI/ROI and interchangeably used herein).
   a. In STE, only the ST interval (FIG. 5) is required to be analyzed. More specifically, FIG. 5, with reference to FIGS. 1 through 4, depicts ECG Schematics; P, Q, R, S and T are the ECG waves; PR, ST, QRS, PR and QT are medically important ECG segments of a cardiac cycle, in accordance with an embodiment of the present disclosure. As depicted in FIG. 5, the rest of the cardiac cycle does not play any role in detecting STE. Lead-wise, V2 and V3 may show some STE and the hence medical experts suggest the following adjustments for both V2 and V3.
      i. For male subjects, > 2 mm is significant.
      ii. For female subjects, > 1.5 mm is significant.
   b. In IAVB, the PR interval (FIG. 5) needs to be more than 200 milliseconds. PR interval can be measured in all twelve ECG leads. However, leads having large, wide P waves and long QRS complexes tend to yield more accurate measurement which is verified by examining multiple simultaneously recorded leads (e.g., refer B. Surawicz et al). Like STE, the rest of the cardiac cycle does not affect this decision. As shown in FIGS. 6A and 7A, SHAP is visibly not working great in detecting ROI. In FIG. 6A, the QRS complex region has extremely high SHAP values, even though QRS segment should not be used for STE determination. The same observation remains true for IAVB, as shown in FIG. 7A. FIGS. 6A and 6B, with reference to FIGS. 1 through 5, depict STE: SHAP time-series values with and without explanation and the black horizontal/spike line depicts the ECG signal, while the grey horizontal/spike line is SHAP of subject ID A0021, in accordance with an embodiment of the present disclosure. FIGS. 7A and 7B, with reference to FIGS. 1 through 6B, depict IAVB: SHAP time-series values with and without explanation; the black horizontal/spike line depicts the ECG signal, while the grey horizontal/spike line is SHAP for subject ID A0039, in accordance with an embodiment of the present disclosure. The system 100 of the present disclosure decides to delve into a detailed frequency-time analysis of these SHAP values in the spectrograms depicted in FIGS. 8A and 9A. FIGS. 8A and 8B, with reference to FIGS. 1 through 7B, depict SHAP spectrogram with and without explanation for STE, in accordance with an embodiment of the present disclosure. Higher frequency components in FIG. 7A are curtailed using a band pass filter, thereby producing FIG. 7B, subject ID A0021. FIGS. 9A and 9B, with reference to FIGS. 1 through 8B, depict SHAP spectrogram with and without explanation for IAVB, in accordance with an embodiment of the present disclosure. Higher frequency components in FIG. 9A are curtailed using a band pass filter, thereby producing FIG. 9B for subject ID A0039. The FIGS. 6A through 9B reconfirm that there is indeed huge energy in the SHAP values, correspondent to the QRS complex of respective cardiac cycles, especially in the higher frequencies. In order to remove those, the system 100 applies a second order Butterworth band pass filter (e.g., filtering technique/thresholding technique as known in the art) with cutoff frequencies of 0.001 Hz and 5 Hz (e.g., cut-off frequency range). As shown in FIG. 3, the system 100 performs a linear normalization on the output of the band pass filter.

Referring to steps of FIG. 4, at step 212 of the method of the present disclosure, the one or more hardware processors 104 normalize the filtered set of contribution values pertaining to the multi-lead ECG signal, to obtain a set of normalized contribution values. The normalization is shown in FIG. 3 (e.g., refer linear normalization block of FIG. 3).

At step 214 of the method of the present disclosure, the one or more hardware processors 104 determine one or more prominent ECG leads based on a comparison of (i) a mean contribution value computed for each lead (also referred to as an average of contribution value of each lead) present in the multi-lead ECG signal using the set of normalized contribution values and (ii) a threshold contribution value. The above step of 214 is better understood by way of following description:

Explainable ECG Lead: All twelve ECG leads are not medically required to classify the diseases. Let us take two specific diseases from that list, to underline this domain-specific lead-dependency.
a. Only V1, V2 and V3 leads (e.g., the one or more prominent ECG leads) are required to conclusively determine the presence of ST Segment Elevation (STE) (e.g., "K. Thygesen, J. S. Alpert, A. S. Jaffe, B. R. Chaitman, J. J. Bax, D. A. Morrow, H. D. White, and E. G. on behalf of the Joint European Society of Cardiology (ESC)/American College of Cardiology (ACC)/American Heart Association (AHA)/World Heart Federation (WHF) Task Force for the Universal Definition of Myocardial Infarction, "Fourth universal definition of myocardial infarction (2018)," Circulation, vol. 138, no. 20, pp. e618-e651, 2018.").
b. As opposed to STE, any of the twelve leads, can potentially determine First-degree Atrioventricular Block (IVAB) (e.g., refer "B. Surawicz and T. Knilans, Chou's electrocardiography in clinical practice: adult and pediatric. Elsevier Health Sciences, 2008. - B. Surawicz et al"). A good explanation should be able to capture this disease-to-lead mapping.

One or more associated region of interests (RoIs) of the one or more prominent ECG leads are then identified based on an associated contribution value for computing the explanation performance.

At step 216 of the method of the present disclosure, the one or more hardware processors 104 analyze a contribution of the one or more prominent ECG leads with respect to a reference lead contributing to a specific disease class amongst the one or more disease classes as classified by the trained classifier. Similarly, the contribution of the one or more associated region of interest (RoI) of the one or more prominent ECG leads is also analysed for computing the explanation performance. The above step of 216 is better understood by way of following description:

As shown in FIG. 3, ECG signals are fed into the ResNet model/classifier, yielding classification for each of those 12 lead signals. The performances of the architecture of Zhang et al. and a lighter implementation of that are compared in Table 1. More specifically, Table 1 illustrates classification of leading state of the art method (e.g., refer "D. Zhang, S. Yang, X. Yuan, and P. Zhang, "Interpretable deep learning for automatic diagnosis of 12-lead electrocardiogram," Iscience, vol. 24, no. 4, 2021.") and a lighter version of the system 100 of FIG. 1.

**Table 1**

| Models | Classification F1-score (%) | Flops (billions) |
|---|---|---|
| Four residual blocks | 83.0 | 12 |
| Two residual blocks | 82.9 | 2.3 |

By reducing to two residual blocks, the number of flops gets reduced by more than five times, with negligible reduction in the F1-score. Hence, one may want to choose the lighter model, over the more complex model by Zhang et al. However, in addition to classification performance, the system 100 explores explanation performance.

At step 218 of the method of the present disclosure, the one or more hardware processors 104 compute an explanation performance with respect to a ground-truth explanation using the analysed contribution of the one or more prominent ECG leads and/or the associated one or more RoIs. In one embodiment of the present disclosure, the explanation performance is computed by categorizing the multi-lead ECG signal into a plurality of categories. For instance, the plurality of categories include a first category indicative of a presence of the one or more prominent ECG leads and the reference lead in the multi-lead ECG signal, a second category indicative of an absence of the one or more prominent ECG leads and the reference lead in the multi-lead ECG signal, a third category indicative of the presence of the one or more prominent ECG leads and the absence of the reference lead in the multi-lead ECG signal, and a fourth category indicative of the absence of the one or more prominent ECG leads and the presence of the reference lead in the multi-lead ECG signal. The correctness of the generated explanation is measured based on number of one or more categories identified amongst the plurality of categories. For instance, the correctness of the generated explanation may be based on the first category (e.g., the presence of the one or more prominent ECG leads and the reference lead in the multi-lead ECG signal). In other instances, the correctness of the generated explanation may be based on other categories as mentioned above. In a further instance, the correctness of the generated explanation may be based on a combination of the categories. The above step of 218 is better understood by way of following description:

### RESULTS:

### Qualitative Explanation:

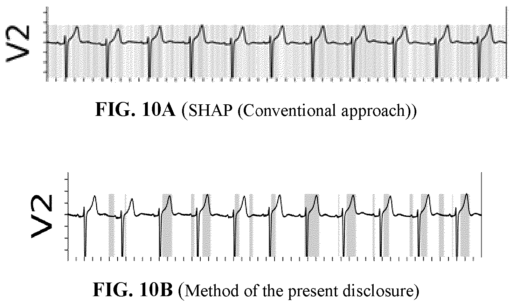

The system 100 provides ROI-based explanation to the primary caregivers in telehealth ecosystem. The system 100 checks explanation qualitatively. The resulting time-series diagrams, post explanations, reveal higher energy in explainable ROIs: ST intervals in STE and PR intervals in IVAB (FIG. 6B and FIG. 7B respectively). The effect is even more evident in the resulting spectrograms. The method of the present disclosures provides explanation comprising the contribution values (which are in the form of vector/matrix) successfully removes high energy regions visible in FIG. 8A and thereby exposes low frequency (sub 10 Hz) energy of the ST interval regions. Similar trends are visible in all six leads selected in IAVB, and PR intervals are the most prominent regions. However, the system 100 and the method of the present disclosure observed and concluded that both these representations, namely, SHAP time-series and SHAP spectrogram (FIGS. 6A through FIG. 9B) are not suitable for quick qualitative explanation. The ease of the qualitative interpretation can be improved if the background for the selected ROIs is highlighted in STE and IAVB. The state of the art (or conventional) methods produced a cluttered background, while the explanation with the contribution values generated by the system 100 produces a much clearer ROI (refer FIGS. 10A and 10B). More specifically, FIGS. 10A and 10B, with reference to FIGS. 1 through 9B, depict qualitative results for STE for subject ID A0021, in accordance with an embodiment of the present disclosure. This brings in qualitative clinical significance and enables physicians (or medical professionals/representatives) to quickly glance through the explanation offered by method of the present disclosure.

### Quantitative Explanation

The ground truth ROI (e.g., reference RoI) is the domain backed ECG segment, medically relevant for the respective disease. Hence, the method to quantify explainability is disease specific. The system 100 checks the explanation ROI against the medical ROI (ground truth), and/or a presence of the one or more prominent ECG leads against a present of the reference ECG lead and calculate True Positive (TP), True Negative (TN), False Positive (FP) and False Negative (FN). Each sample of ECG is categorized as either TP, TN, FP and FN in the following manner.
1. First category (TP) = the one or more prominent ECG leads/prominent ROI and medical/reference lead/ROI, both present.
2. Second category (TN) = the one or more prominent ECG leads/prominent ROI and medical/reference lead/ROI, both absent.
3. Third category (FP) = the one or more prominent ECG leads/prominent ROI present, but the medical/reference lead/ROI absent.
4. Fourth category (FN) = the one or more prominent ECG leads/prominent ROI absent, but the medical/reference lead/ROI present.

The system 100 calculated accuracy and F1-score, for two DL models of varied complexities and hence, varying floating point operations (flops). As depicted in Table 2, the more complex model provides better explainability (F1-score is 35.8%) for STE test dataset. Hence, four residual blocks are justified as a better operating model, despite having more than 5x time complexities. However, while running the method of the present disclosure on IVAB test set, a different trend has been observed. The lighter model seems to be providing better explainability (F1-score is 40.6%) in IAVB. This can be explained as follows: the manifestation of IAVB is theoretically visible in all twelve ECG leads. Hence, complex models are not required to explain IAVB. This conjecture is further supported by the fact that the difference in classification performance of four residual blocks and two residual blocks is negligible (91%). Hence, in summary, the two residual block model provides better explainability performance with similar classification performance, while using less than 20% flops. Therefore, as an operating DL model, one may want to choose the lighter model, over the four-residual-block architecture in IAVB classification. Below Table 2 illustrates classification and explanation results for STE and Table 3 illustrates classification and explanation results for IAVB.

**Table 2**

| Models | Classification [F1-score] | Flops | Explainability [F1-score, Accuracy] | |
|---|---|---|---|---|
| | | | SHAP | Method of the present disclosure |
| 4 residual blocks | 80.0 | 12 B | 26.1, 50.7 | **35.8,** 70.5 |
| 2 residual blocks | 77.55 | 2.3 B | 25.5, 50.4 | 26.3, 76.5 |

**Table 3**

| Models | Classification [F1-score] | Flops | Explainability [F1-score, Accuracy] | |
|---|---|---|---|---|
| | | | SHAP | Method of the present disclosure |
| 4 residual blocks | 91.54 | 12 B | 37.7, 48.5 | 32.5, 66.2 |
| 2 residual blocks | 91.03 | 2.3 B | 39.2, 49.8 | 40.6, 71.8 |

As depicted in Table 2, the 2 residual blocks model provides similar explainability/explanation performance with respect to the 2 residual blocks model. Though there is slight difference in the classification accuracy, the explanation performance is similar. This difference enables the system 100 to choose the 2 residual blocks model (DL model) over the 4 residual blocks model (DL model) since the floating operations (flops) is 12 billion the 4 residual blocks model (DL model) as compared to 2.3 billion for the 2 residual blocks model (DL model). Hence, it is evident that the time complexity is more for using the 4 residual blocks model (DL model) and reduced time complexity in using the 2 residual blocks model (DL model). This gives an advantage to the system 100 to observe the performance and execution and dynamically select appropriate DL model. It is to be understood by a person having ordinary skill in the art or person skilled in the art that depending on the application and requirement of the implementation of the system 100 and the method of the present disclosure, the steps from 208 through 218 may also be executed by the explainer model and/or the classification model as implemented herein, and such execution and implementation of the above mentioned models shall not be construed as limiting the scope of the present disclosure.

### Two use-cases:

The system 100 used two subjects - ID A0021 and ID A0039, as use-cases of STE and IAVB respectively. It is to be noted that similar explanations can be extended to other STE and IAVB subjects of CPSC2018DB. Tables 4 and 5 compare lead-wise quantification performance of SHAP and explanation method of the present disclosure.

**Table 4 (lead-wise result for STE; subject ID A0021)**

| Lead | F1-score | | Accuracy | |
|---|---|---|---|---|
| | SHAP | Method of the present disclosure | SHAP | Method of the present disclosure |
| V2 | 37.4 | 58.9 | 51.3 | 80.5 |

**Table 5 (lead-wise result for IAVB; subject ID A0039)**

| Lead | F1-score | | Accuracy | |
|---|---|---|---|---|
| | SHAP | Method of the present disclosure | SHAP | Method of the present disclosure |
| I | 39.4 | 61.0 | 51.0 | 84.2 |
| II | 43.2 | 68.1 | 56.4 | 85.7 |
| aVR | 42.1 | 54.6 | 52.6 | 85.1 |
| V1 | 33.8 | 0 | 50.3 | 78.6 |
| V4 | 30.2 | 1.5 | 47.1 | 75.1 |
| V6 | 34.0 | 38.3 | 49.1 | 78.0 |

It is noted and known by the system 100 and the method of the present disclosure that lead requirements of STE and IAVB are different. Table 4 shows that only one ECG lead (V2, medically relevant for STE) got selected for ID A0021. On the other hand, six leads got selected for IAVB (ID A0039). Further, for subject ID A0021, both F1-score and accuracy of the quantification improved greatly from SHAP to the method of the present disclosure. For ID A0039, it can be seen that F1-score and accuracy improved from SHAP to the method of the present disclosure for most of the leads.

The system 100 implemented the method of the present disclosure using 12-lead ECG. A Region of Interest (ROI) based quantitative measure of explanation is provided by way of the method of the present disclosure. Then, the system 100 introduced post-processing steps to SHapley Additive exPlanations (SHAP) to make it better, both qualitatively and quantitatively. The accuracy and F1-score of explainability in STE increased from 50.7% to 70.5% and 26.1% to 35.8% respectively. In IAVB, the improvements for accuracy and F1-score are from 39.2% to 40.6% and 49.8% to 71.8% respectively. Finally, the system 100 demonstrated that the explanation comprising the contribution values provides a unique opportunity to choose an optimal DL architecture, according to the available computing resource and desired level of explanation.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method, comprising:
receiving, via one or more hardware processors, a multi-lead electrocardiogram (ECG) signal associated with a user (202);
classifying, by using a trained classifier via the one or more hardware processors, the multi-lead ECG signal into one or more disease classes based on information present in each lead in the multi-lead ECG signal (204);
generating, by using the trained classifier via the one or more hardware processors, an explainer model, based on an associated ECG training dataset (206);
generating, by using the explainer model via the one or more hardware processors, an explanation comprising a contribution value to each data point present in each lead of the multi-lead ECG signal (208), wherein the contribution value is computed using the trained classifier and the multi-lead ECG signal;
obtaining, via the one or more hardware processors, a filtered set of contribution values pertaining to a plurality of data points present in the multi-lead ECG signal based on a comparison of (i) the contribution value of the generated explanation assigned to each data point present in the multi-lead ECG signal, and (ii) a cut-off frequency range (210);
normalizing, via the one or more hardware processors, the filtered set of contribution values pertaining to the multi-lead ECG signal, to obtain a set of normalized contribution values (212);
determining, via the one or more hardware processors, one or more prominent ECG leads based on a comparison of (i) a mean contribution value computed for each lead present in the multi-lead ECG signal using the set of normalized contribution values and (ii) a threshold contribution value (214);
analyzing, via the one or more hardware processors, a contribution of the one or more prominent ECG leads with respect to a reference lead contributing to a specific disease class amongst the one or more disease classes as classified by the trained classifier (216); and
computing, via the one or more hardware processors, an explanation performance with respect to a ground-truth explanation using the analysed contribution of the one or more prominent ECG leads (218).

2. The processor implemented method as claimed in claim 1, wherein the explanation performance is computed by categorizing the multi-lead ECG signal into a plurality of categories.

3. The processor implemented method as claimed in claim 2, wherein the plurality of categories comprises a first category indicative of a presence of the one or more prominent ECG leads and the reference lead in the multi-lead ECG signal, a second category indicative of an absence of the one or more prominent ECG leads and the reference lead in the multi-lead ECG signal, a third category indicative of the presence of the one or more prominent ECG leads and the absence of the reference lead in the multi-lead ECG signal, and a fourth category indicative of the absence of the one or more prominent ECG leads and the presence of the reference lead in the multi-lead ECG signal, and wherein correctness of the generated explanation is measured based on number of one or more categories identified amongst the plurality of categories.

4. The processor implemented method as claimed in claim 1, wherein a higher contribution value is assigned to the one or more prominent ECG leads in the multi-lead ECG signal which contributed to a classification of a disease to a desired disease class amongst the one or more disease classes.

5. The processor implemented method as claimed in claim 1, wherein the filtered set of contribution values is obtained by applying a thresholding technique on the contribution value assigned to each data point present in the multi-lead ECG signal using the cut-off frequency range.

6. The processor implemented method as claimed in claim 1, further comprising identifying an associated region of interest (RoI) of the one or more prominent ECG leads based on an associated contribution value for computing the explanation performance.

7. A system (100), comprising:
a memory (102) storing instructions;
one or more communication interfaces (106); and
one or more hardware processors (104) coupled to the memory (102) via the one or more communication interfaces (106), wherein the one or more hardware processors (104) are configured by the instructions to:
receive a multi-lead electrocardiogram (ECG) signal associated with a user;
classify, by using a trained classifier, the multi-lead ECG signal into one or more disease classes based on information present in each lead in the multi-lead ECG signal;
generate, by using the trained classifier, an explainer model, based on an associated ECG training dataset;
generate, by using the explainer model, an explanation comprising a contribution value to each data point present in each lead of the multi-lead ECG signal, wherein the contribution value is computed using the trained classifier and the multi-lead ECG signal;
obtain a filtered set of contribution values pertaining to a plurality of data points present in the multi-lead ECG signal based on a comparison of (i) the contribution value of the generated explanation assigned to each data point present in the multi-lead ECG signal, and (ii) a cut-off frequency range;
normalize the filtered set of contribution values pertaining to the multi-lead ECG signal, to obtain a set of normalized contribution values;
determine one or more prominent ECG leads based on a comparison of (i) a mean contribution value computed for each lead present in the multi-lead ECG signal using the set of normalized contribution values and (ii) a threshold contribution value;
analyze a contribution of the one or more prominent ECG leads with respect to a reference lead contributing to a specific disease class amongst the one or more disease classes as classified by the trained classifier; and
compute an explanation performance with respect to a ground-truth explanation using the analysed contribution of the one or more prominent ECG leads.

8. The system as claimed in claim 7, wherein the explanation performance is computed by categorizing the multi-lead ECG signal into a plurality of categories.

9. The system as claimed in claim 8, wherein the plurality of categories comprises a first category indicative of a presence of the one or more prominent ECG leads and the reference lead in the multi-lead ECG signal, a second category indicative of an absence of the one or more prominent ECG leads and the reference lead in the multi-lead ECG signal, a third category indicative of the presence of the one or more prominent ECG leads and the absence of the reference lead in the multi-lead ECG signal, and a fourth category indicative of the absence of the one or more prominent ECG leads and the presence of the reference lead in the multi-lead ECG signal, and wherein correctness of the generated explanation is measured based on number of one or more categories identified amongst the plurality of categories.

10. The system as claimed in claim 7, wherein a higher contribution value is assigned to the one or more prominent ECG leads in the multi-lead ECG signal which contributed to a classification of a disease to a desired disease class amongst the one or more disease classes.

11. The system as claimed in claim 7, wherein the filtered set of contribution values is obtained by applying a thresholding technique on the contribution value assigned to each data point present in the multi-lead ECG signal using the cut-off frequency range.

12. The system as claimed in claim 7, wherein the one or more hardware processors are further configured by the instructions to identify an associated region of interest (RoI) of the one or more prominent ECG leads based on an associated contribution value for computing the explanation performance.

13. One or more non-transitory machine-readable information storage mediums comprising one or more instructions which when executed by one or more hardware processors cause:
receiving a multi-lead electrocardiogram (ECG) signal associated with a user;
classifying, by using a trained classifier, the multi-lead ECG signal into one or more disease classes based on information present in each lead in the multi-lead ECG signal;
generating, by using the trained classifier, an explainer model, based on an associated ECG training dataset;
generating, by using the explainer model, an explanation comprising a contribution value to each data point present in each lead of the multi-lead ECG signal, wherein the contribution value is computed using the trained classifier and the multi-lead ECG signal;
obtaining a filtered set of contribution values pertaining to a plurality of data points present in the multi-lead ECG signal based on a comparison of (i) the contribution value of the generated explanation assigned to each data point present in the multi-lead ECG signal, and (ii) a cut-off frequency range;
normalizing the filtered set of contribution values pertaining to the multi-lead ECG signal, to obtain a set of normalized contribution values;
determining one or more prominent ECG leads based on a comparison of (i) a mean contribution value computed for each lead present in the multi-lead ECG signal using the set of normalized contribution values and (ii) a threshold contribution value;
analyzing a contribution of the one or more prominent ECG leads with respect to a reference lead contributing to a specific disease class amongst the one or more disease classes as classified by the trained classifier; and
computing an explanation performance with respect to a ground-truth explanation using the analysed contribution of the one or more prominent ECG leads.

14. The one or more non-transitory machine-readable information storage mediums as claimed in claim 13, wherein the explanation performance is computed by categorizing the multi-lead ECG signal into a plurality of categories, and wherein the plurality of categories comprises a first category indicative of a presence of the one or more prominent ECG leads and the reference lead in the multi-lead ECG signal, a second category indicative of an absence of the one or more prominent ECG leads and the reference lead in the multi-lead ECG signal, a third category indicative of the presence of the one or more prominent ECG leads and the absence of the reference lead in the multi-lead ECG signal, and a fourth category indicative of the absence of the one or more prominent ECG leads and the presence of the reference lead in the multi-lead ECG signal, and wherein correctness of the generated explanation is measured based on number of one or more categories identified amongst the plurality of categories.

15. The one or more non-transitory machine-readable information storage mediums as claimed in claim 13,
wherein a higher contribution value is assigned to the one or more prominent ECG leads in the multi-lead ECG signal which contributed to a classification of a disease to a desired disease class amongst the one or more disease classes,
wherein the filtered set of contribution values is obtained by applying a thresholding technique on the contribution value assigned to each data point present in the multi-lead ECG signal using the cut-off frequency range, and
wherein the one or more instructions which when executed by the one or more hardware processors further cause identifying an associated region of interest (RoI) of the one or more prominent ECG leads based on an associated contribution value for computing the explanation performance.
